# EUROPÄISCHE PATENTSCHRIFT

(11) **EP 3 651 680 B1**
(45) Veröffentlichungstag und Bekanntmachung des Hinweises auf die Patenterteilung: **20.07.2022**
(21) Anmeldenummer: 18727772.8
(22) Anmeldetag: 24.05.2018
(51) Int. Cl.: A61C 1/08, A61C 3/02, C09K 11/77, F21K 9/64, H01L 33/58, A61B 1/06, H01L 33/54, H01L 33/50, H01L 33/48, A61B 90/30, A61B 1/24, F21Y 115/10

(54) **LED/LASER-BELEUCHTUNGSVORRICHTUNG MIT GETRENNTER LEUCHTSTOFFKONFIGURATION UND VERFAHREN ZUR HERSTELLUNG EINER SOLCHEN**
LED/LASER ILLUMINATION DEVICE WITH A REMOTE PHOSPHOR CONFIGURATION AND PRODUCTION METHOD FOR SUCH A DEVICE
DISPOSITIF D'ILLUMINATION À DEL OU LASER AVEC PHOSPHORE SÉPARÉ ET MÉTHODE DE FABRICATION D'UN TEL DISPOSITF

(30) Priorität: 13.07.2017 DE 102017212030
(43) Veröffentlichungstag der Anmeldung: 20.05.2020
(73) Patentinhaber: Tridonic GmbH & Co KG, 6851 Dornbirn (AT); W & H Dentalwerk Bürmoos GmbH, 5111 Bürmoos (AT)
(72) Erfinder: RIEMER, Steffen, 8564 Krottendorf-Gaisfeld (AT); SCHRANK, Franz, 8074 Raaba (AT); UITZ, Patrick, 8350 Fehring (AT); BRUGGER, Wilhelm, 5071 Wals-Siezenheim (AT); IRRAN, Thomas, 5020 Salzburg (AT)
(74) Vertreter: Banzer, Hans-Jörg
(86) Internationale Anmeldenummer: PCT/EP2018/063672
(87) Internationale Veröffentlichungsnummer: WO 2019/011511

(56) Entgegenhaltungen:
- DE-A1-102005 023 134
- DE-A1-102015 103 331
- DE-U1-202005 020 763
- US-A1- 2014 140 071
- US-A1- 2016 139 300
- Schott AG: "Die erste ringförmige autoklavierbare High Brightness LED von SCHOTT", , 9. Februar 2015 (2015-02-09), XP055290210, www.schott.com Gefunden im Internet: URL:http://www.schott.com/newsfiles/com/01 3_2015_schott-ring-leds-for-dental-and-med ical-applications_de_final.pdf [gefunden am 2016-07-21]

## Beschreibung

Die Erfindung betrifft eine Beleuchtungsvorrichtung auf der Basis von LED's oder Laserdioden (LD's) mit einer neuartigen hermetisch dichten Remote-Leuchtstoff-Konfiguration in Form eines optischen Elementes (z.B. einer Linse) aus einem Glasmaterial, dessen Volumen Lumineszenzmaterial aufweist und als solche für eine Heißdampfsterilisation geeignet ist sowie ein Verfahren zur Herstellung einer solchen, wobei im Folgenden unter Leuchtstoff lumineszierende Materie verstanden wird.

Vorbekannte Beleuchtungsvorrichtungen mit Remote-Leuchtstoff-Konfigurationen dieser Art sind miniaturisierte LED-Module, die auch in ringförmiger Anordnung, beispielsweise in medizinischen Instrumenten wie medizinischen, insbesondere dentalen Hand- oder Winkelstücken, Verwendung finden. Hermetisch dichte Ausführungen werden beispielsweise in folgender Ausführung hergestellt (siehe DE102015103331B4):
Auf einer Basis (einem Sockel bzw. Halter, im weiteren Verlauf der Beschreibung Substrat genannt) aus Keramik oder Metall werden blau emittierende LED- Chips montiert und ein Abstandshalter in Form einer Kappe aus Metall mit einem bzw. mehreren Fenstern aus transparentem Glas darauf verlötet oder verschweißt. Vor der Verbindung mit dem Substrat jedoch wird ein Konverter als dünn ausgeführtes Element auf der Innenseite des transparenten Fensters verklebt. Das Prinzip ist dabei stets, dass ein Luftraum an den LEDs angrenzt, eine Remote-Leuchtstoff-Folie beabstandet angeordnet ist, über der wiederum eine transparente Linse angeordnet ist.

Im Falle dass im Stand der Technik Leuchtstoffpartikel in einer Matrix aus Glas beschrieben werden, handelt es sich ausschließlich um aufgebrachte, geklebte oder gesinterte Schichten auf einem Träger aus Glas (oder aus Keramik), die allesamt nicht für die Herstellung einer hermetisch dichten LED-Anordnung geeignet sind.

In US20120107622A1 beispielsweise wird die Leuchtstoffschicht in einer Art Sinterprozess auf eine Glasplatte aufgebracht.

In US20080029720A1 ist eine Leuchtstoffschicht in organischem Bindemittel beschrieben, ähnlich einer Lackierung oder Beschichtung.

WO2006097876 offenbart Leuchtstoffe in einer keramischen Matrix. WO2014178515A1 betrifft beschichtete Formteile aus Glas, Leuchtstoffpartikel ähnlich wie den Dokumenten zuvor, in einer Matrix aus Glas.

In US20110215701 werden im Rahmen der Beschichtung eines Trägers mit Leuchtstoff verschiedene Bindemittel und lichtstreuende Zusätze angeführt. US20100002450A1 beschreibt einen Linsenhalter auf Basis einer mechanischen Fixierung. Farbkonversion oder Leuchtstoff ist in diesem Dokument nicht erwähnt.

Der Prozess zur Erzielung einer hermetisch dichten Anordnung ausgehend von Beleuchtungsvorrichtungen der oben aufgeführten Bauarten ist zeitaufwendig, teuer und nach wie vor mit Problemen behaftet. So kann beispielsweise die Verklebung des Konverters auf dem Fenster versagen oder die Bauhöhe des Abstandhalters (der metallischen Kappe) kann nicht weiter vermindert werden, weil der Konverter (das Konverterelement) typischerweise 200 µm dick ist. Sämtliche im Stand der Technik angegebene Lösungen, die dispergierte Leuchtstoffpartikel in einer Matrix aus Glas oder Keramik beschreiben, erweisen sich als ungünstig für die Herstellung einer hermetisch dichten Verbindung mit einem metallischen Formkörper.

Zusammengefasst ergeben Verklebungen oder Beschichtungen mit Kunststoffen - derzeit der Stand der Technik auf diesem Gebiet - nicht die erfindungsgemäße dichte Ausführung, die für eine gewünschte und im medizinischen Einsatz geforderte Heißdampfsterilisation und Temperaturschock-Beständigkeit notwendig ist.

Der Erfindung liegt daher die Aufgabe zugrunde eine Beleuchtungsvorrichtung dieser Art zu vereinfachen, um somit eine robuste und zuverlässige Ausführung für das Remote Leuchtstoffelement in hermetisch dichter Bauweise zu schaffen, geeignet für die Heißdampfsterilisation für die medizinische Anwendung, sowie ein Verfahren anzugeben, wie eine solche Vorrichtung einfach und kostengünstig hergestellt werden kann.

Diese Aufgabe wird durch die Merkmale der unabhängigen Ansprüche gelöst. Vorteilhafte Weiterbildungen der Erfindung sind in den zugehörigen Unteransprüchen beschrieben.
Figur 1 zeigt die erfindungsgemäße Vorrichtung in einer Schnitt-Ansicht A-A.
Figur 2 zeigt die Draufsicht auf eine solche Vorrichtung.
Figur 3 zeigt ein medizinisches Instrument in Form eines dentalen Hand- oder Winkelstücks.
Figur 4 zeigt den vorderen Teil des medizinischen Instrumentes mit einer singulären Beleuchtungsvorrichtung.
Figur 5 zeigt das Kopfende eines dentalen Hand- oder Winkelstücks mit einer ringförmigen Beleuchtungsvorrichtung in einer Schnitt-Ansicht B-B.
Figur 6 zeigt die Draufsicht auf das Kopfende des dentalen Hand- oder Winkelstücks mit der ringförmigen Beleuchtungsvorrichtung.

Der Grundgedanke der vorliegenden Erfindung ist es, die bisher getrennte Anordnung von optischem Bauteil (z.B. transparenter Linse) und Remote-Leuchtstoff-Folie in einem einzigen Bauteil zu vereinen, sodass also der Leuchtstoff in das Glasmaterial des optischen Bauelements (z.B. eine Linse) dispergiert, d.h. mit diesem homogen verschmolzen ist, was nicht nur zu besseren thermischen Eigenschaften führt sondern sich generell als stabiler und haltbarer erweist.

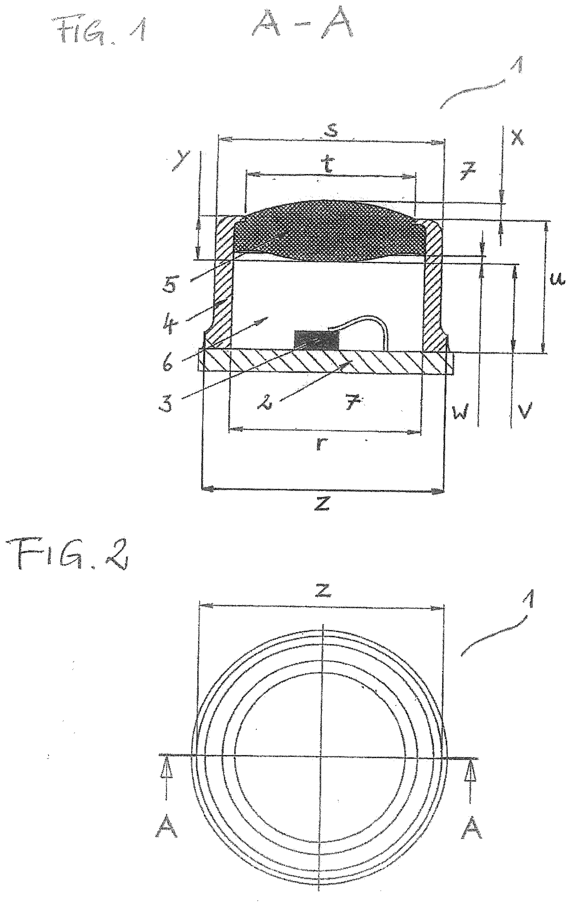

In Figur 1 ist eine Schnittansicht A-A der erfindungsgemäßen Beleuchtungsvorrichtung 1 dargestellt. Figur 2 zeigt eine Draufsicht auf diese Vorrichtung.

Ein vorzugsweise nicht transparentes Substrat 2, beispielsweise aus Keramik, bildet die Basis für die erfindungsgemäße Beleuchtungsvorrichtung 1. Auf diesem Substrat 2 ist das Leuchtelement 3 - ein LED-Element oder eine Laserdiode - fest montiert. Das Leuchtelement 3 kann dabei als Integrierter Schaltkreis oder als SMD-Bauteil (oberflächen montiertes Element, engl. ***S**urface **M**ounted **D**evice*) ausgestaltet sein. Die elektrischen Anschlüsse des Leuchtelementes 3 sind hermetisch dicht durch das Substrat geführt, beispielsweise durch eine in die Keramik miteingebrannte metallische Durchkontaktierung oder durch eine verschmolzene Glasdurchführung. Auf dem Substrat 2 befindet sich ferner ein nichttransparenter Abstandhalter 4 der in einem Abstand zur Substratfläche in einer Öffnung eine Linse oder eine Diffuserscheibe (eine Streuscheibe) oder ein anderes optisches Element 5, was im Folgenden als solches bezeichnet wird, trägt. Der Abstand ist so bemessen, dass ein Luftraum 6 zwischen dem optischen Element und dem Substrat 2 gebildet ist, der das Leuchtelement 3 problemlos aufnehmen kann. Der Abstandhalter 4 ist vorteilhaft aus Metall und zylindrisch und bildet zusammen mit dem von ihm getragenen optischen Element 5 eine Kappe, die das Leuchtelement 3 gegenüber dem Außenraum 7 hermetisch dicht verschließt. Diese für die Erfindung außerordentlich wichtige hermetische Dichtigkeit des Luftraumes 6 gegenüber dem Außenraum 7 wird einerseits durch ein Verlöten oder Verschweißen des Abstandhalters 4 mit dem Substrat 2 erreicht, andererseits durch ein Verschmelzen bzw. Vergießen des optischen Elementes 5 mit dem Abstandshalter 4 im Bereich der Öffnung bei sehr hoher Temperatur. Die ebenfalls hermetische Dichtigkeit der elektrischen Anschlüsse des Leuchtelementes 3 durch das Substrat 2 hindurch wurde bereits beschrieben.

Weiterhin wesentlich für die vorliegende Erfindung ist die Integration des bisher eigenständigen Konverterelementes in das optische Element 5, wodurch einerseits die Komplexität der Vorrichtung reduziert, andererseits die Zuverlässigkeit - insbesondere hinsichtlich Autoklavierbarkeit und Temperatur-Schock-Beständigkeit - deutlich erhöht wird. Der zeitaufwendige Montageschritt für ein Farbkonversionselement entfällt. Anstatt eines bisher verwendeten transparenten, farblosen Glases wird als optisches Element 5 ein Glas-Leuchtstoff-Verbundwerkstoff eingesetzt (wie gesagt ist unter "Leuchtstoff" in diesem Zusammenhang lumineszierende/leuchtende Materie zu verstehen). Dazu wird bei dem im Folgenden beschriebenen Herstellungsverfahren das Glaspulver mit den Leuchtstoffpartikeln homogen gemischt und mit der Öffnung des Abstandhalters verschmolzen. Weiterhin wesentlich für die Erfindung ist, dass der Glasverbundwerkstoff Füllmaterialien zur Einstellung einer Viskosität einer Glasmatrix bei einer Herstellung der Beleuchtungsvorrichtung 1 enthält.

Bei Bedarf können auch weitere lichtstreuende Zusatzstoffe (z.B. Al₂O₃ oder TiO₂) oder weitere Hilfsstoffe beigemischt werden, letzteres um beispielsweise in weiteren Prozeßschritten eine Trennung von Farbstoffteilchen von der Glasmatrix zu verhindern.

Die bevorzugte Ausführungsform der Erfindung ist jedoch die reine homogene Dispersion der Leuchtstoffpartikel im Glasmaterial des optischen Elements 5.

Das Herstellungsverfahren für die eben beschriebene Beleuchtungsvorrichtung 1 ist wie folgt:
Zunächst wird eine hermetisch dichte Verbindung zwischen Abstandhalter 4 und Glasverbundwerkstoff-optisches Element 5 hergestellt. Dieser Schritt kann auf zweierlei Weise erfolgen:

### Variante 1:

Durch Schmelzen des Glaspulvers mit den darin homogen eingemischten Leuchtstoff-Partikeln und eventuell weiteren Zusatzstoffen wird ein stabförmiger Verbund-Glaskörper erzeugt.

Von diesem Glaskörper werden Glasscheiben in einer gewünschten Dicke abgetrennt. Die abgetrennte Glasscheibe wird in die Öffnung des Abstandhalters 4 eingelegt und bei ca. 1000°C (900-1200 °C) mit diesem im Bereich der Öffnung unter Schutzgas dicht verschmolzen bzw. vergossen.

### Variante 2:

Das Glaspulver wird mit den darin homogen eingemischten Leuchtstoff-Partikeln und eventuell weiteren Zusatzstoffen zu einer Tablette gepresst.

Diese Tablette wird in die Öffnung des Abstandhalters 4 eingelegt und bei ca. 1000°C (900-1200 °C) mit diesem im Bereich der Öffnung unter Schutzgas dicht verschmolzen bzw. vergossen.

Beispielsweise kann sich dabei durch die Oberflächenspannung des flüssigen Glasverbundes eine Linsenform des optischen Elementes 5 ergeben. Bei beiden Varianten können aber auch durch geeignete Maßnahmen (z.B. durch Formschalen) andere (Linsen-) Formen erzielt werden.

Anschließend wird das Leuchtelement 3 auf dem Substrat 2 hermetisch dicht angebracht und die elektrischen Zuleitungen des Leuchtelementes 3 durch das Substrat 2 mittels Glasdurchführung oder metallischer Durchkontaktierung hermetisch abgedichtet.

Zuletzt wird das Substrat 2 mit dem Abstandhalter 4 durch Löten oder Schweißen (bei zu unterschiedlichen schwer zu verbindenden Materialien - z.B. Metall/Keramik - vorzugsweise durch Laserschweißen) ebenfalls hermetisch dicht verbunden.

Es soll nochmals betont werden, dass durch die oben beschriebene Vorgehensweise die Leuchtstoffpartikel im Volumen des einteilig vergossenen optischen Elementes (z.B. einer Glaslinse) ausschließlich homogen verteilt sind, im Gegensatz zu einem populären Stand der Technik-Verfahren, bei dem eine Leuchtstoffschicht auf einem Glaskörper aufgesintert wird (beschrieben in der US 20120107622A1).

Ebenso wenig sind Klebeverbindungen notwendig (z.B. das Aufkleben einer Leuchtstoff-Folie auf das optische Element im Stand der Technik, die Folie als jenes Bauteil, welches am wenigsten temperaturstabil ist) sowie das Anbringen eines vorgefertigten Leuchtstoff/Glaskörpers ohne nachträglichen Einschmelzvorgang, was die gewünschte Thermostabilität der Vorrichtung erheblich schmälern würde. Die oben erwähnte Möglichkeit der Verwendung einer leistungsstärkeren Laserdiode (LD) als Leuchtelement 3 ist ebenfalls nur aufgrund der hohen Thermostabilität des gesamten Systems möglich.

Da der Schmelzvorgang bzw. Einschmelzvorgang des Leuchtstoffglaskörpers relativ hohe Temperaturen erfordert, die gegebenenfalls die Effizienz des Leuchtstoffs beeinträchtigen können, werden Leuchtstoffe vorgeschlagen, die sehr temperaturstabil sind und sich deswegen für die Erfindung besonders gut eignen. Diese sind gelb und grün emittierende Granate wie YAG: Ce³⁺, LuAG. Ce³⁺, rot emittierende Oxide wie Y₂O₃:

Eu³⁺, oder Oxynitrid/Nitrid basierte rote Leuchtstoffe (z.B. SrAlSi₄N₇:E_{U}²⁺, (Ca, Sr)AlSi₄N₇:Eu²⁺, CaSiAlON:Eu²⁺, CaAlSi(ON)₃:Eu²⁺, CaAlSiN₃:Eu oder (Ca, Sr)AlSiN₃:Eu) mit einer homogenen Volumenverteilung von 5 bis 40 Vol.% , vorzugsweise aber zwischen 10 und 25 Vol.% in Einzel- oder Gesamtkonzentration. Abhängig von der gewünschten Farbtemperatur des emittierten Weißlichtes können Leuchtstoffmischungen mit 2 oder 3 Leuchtstoffen unter Berücksichtigung der erwähnten Gesamtkonzentrationen eingesetzt werden. Vorteilhafterweise sollte die Gesamtkonzentration der Leuchtstoffe von 40.Vol% nicht überschritten werden. Sphärische (kugelförmige) Partikel mit glatter Oberfläche sind bei dieser Anwendung besonders vorteilhaft. Die Durchschnittskorngröße der angewendeten Leuchtstoffe (der sogenannte d₅₀-Wert) liegt zwischen 2-20 µm, bevorzugt zwischen 5-18 µm.

Bei einer zylindrisch ausgebildeten Beleuchtungsvorrichtung 1 der vorliegenden Erfindung - im Hinblick auf eine Verwendung in der Zahnmedizin oder Endoskopie - und einer Linse als optisches Element 5 sind folgende Größenordnungen vorgesehen:
(r) Durchmesser Linse=Innendurchmesser Abstandhalter: 0,5-10,0 mm z.B. 1,73 (+0,04;-0,01) mm;
(s) Außendurchmesser Abstandhalter: 1,0-15 mm z.B. 2,05 (+0,02;-0,05) mm;
(t) Durchmesser Öffnung: 0,5-10,0 mm z.B. 1,53 mm;
(u) Höhe Abstandhalter: 0,5-5 mm z.B. 1,20 (±0,10) mm;
(v) Höhe Luftraum: 0,01-4,8 mm z.B. 0,70 (+0,17; 0,00) mm;
(w) Linsenwölbung Innen: 0- +/- 5 mm z.B. 0,07 mm maximal;
(x) Linsenwölbung Außen: 0- +/- 5 mm z.B. 0,15 (0,00;-0,20) mm;
(y) Linsenwölbung Innen bis Oberkante Abstandhalter: z.B. 0,40 (0,00;-0,10) mm;
(z) Durchmesser obere Außenkante Schweißnut/Lötnut: 1,0-15 mm z.B. 2,20 (±0,10) mm.

Ferner haben Untersuchungen im Rahmen der Entwicklung der erfindungsgemäßen Beleuchtungsvorrichtung ergeben, dass bei obiger Bemaßung eine Dicke des Glasverbundwerkstoffes zwischen 0,2 und 1,0 mm, vorzugsweise zwischen 0,4 und 0,8 mm optimal erscheint.

Bei einer derartigen Bemaßung der erfindungsgemäßen Beleuchtungsvorrichtung 1 und hermetischen Abdichtung gemäß des oben beschriebenen Verfahrens, können im Luftraum der Beleuchtungsvorrichtung Leckraten von weniger als 10⁻⁸ mbar-Liter/Sekunde erreicht werden, was - wie bereits erwähnt - eine stabile Autoklavierbarkeit und Temperatur-Schock-Beständigkeit ermöglicht. Ausführungen in anderen Größenordnungen (beispielsweise im Submillimeter-Bereich oder im Zentimeterbereich) für andere Anwendungen und Einsatzgebiete sind nicht ausgeschlossen.

Die Autoklavierbarkeit, d.h. die Eignung für Heißdampfsterilisation sowie eine hohe Temperaturschock-Beständigkeit sollte auch dadurch gewährleistet sein, dass die Materialien aller Komponenten der erfindungsgemäßen Vorrichtung sowie deren Verbindungen untereinander entsprechend gewählt bzw. beschaffen sind.

Die Beleuchtungsvorrichtung 1 kann ebenso als Ringlicht oder in Form verwandter Bauformen ausgebildet sein, wobei das transparente optische Element 5 aus lumineszierenden Glasverbundwerkstoff eine einseitige oder eine beidseitige Krümmung aufweisen kann.

Dementsprechend kann der Abstandhalter 4 mehrere (möglicherweise radial symmetrisch angeordnete unterschiedlich große) Öffnungen aufweisen die jeweils durch ein geeignetes optisches Element 5 hermetisch dicht verschlossen sind und wobei zu einer jeden Öffnungs-optisches Element-Kombination ein auf dem Substrat hermetisch dicht befestigtes Leuchtelement 3 korrespondiert, und wobei die optischen Elemente 5 und die Leuchtelemente 3 der verschiedenen Öffnungen durchaus unterschiedlich sein können.

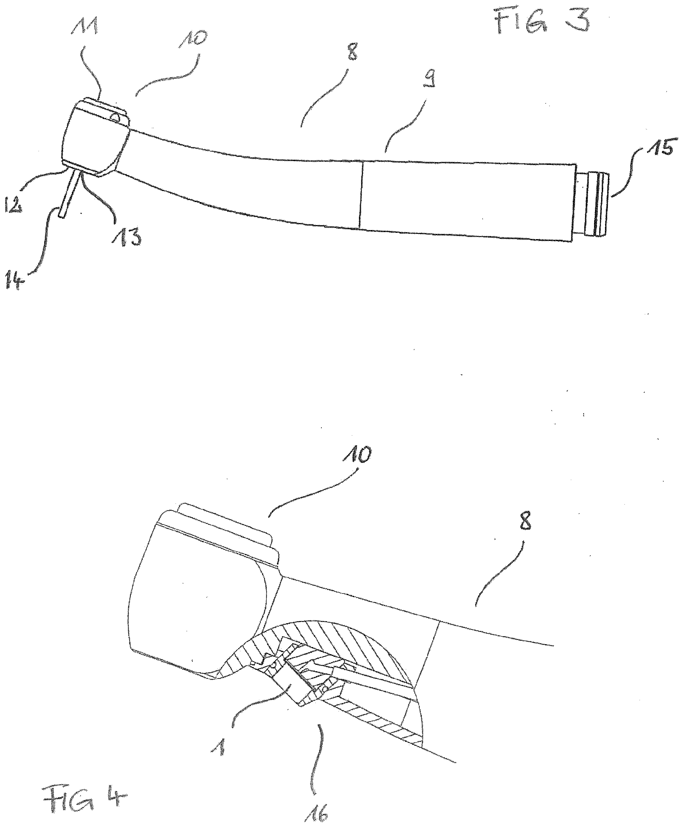

In den Figuren 3 bis 6 soll gezeigt werden, wie die erfindungsgemäße Beleuchtungsvorrichtung 1 beispielsweise in der Dentaltechnik eingesetzt werden kann.

Das medizinische, insbesondere dentale Handstück 8 umfasst ein Griffteil 9 und einen gewinkelt dazu angeordnetes Kopfteil 10. Im Kopfteil 10 ist eine in Bewegung versetzbare, vorzugsweise rotierende Werkzeugaufnahme bzw. Werkzeugöffnung 13 angeordnet, in der ein Werkzeug 14, zum Beispiel ein in Rotation versetzbarer Bohrer, aufgenommen werden kann. Dieser kann beispielsweise durch Drücken eines Drücktasters 11 entriegelt und wieder entfernt werden.

Im Falle von Figur 3 steht das Werkzeug 14 somit gewinkelt zur Längsachse des Griffteils 9 aus dem Kopfteil 10 heraus. Das Werkzeug 14 wird durch zumindest ein Antriebselement angetrieben und ist als solches mit dem Werkzeug 14 verbunden. In diesem Fall ist das Antriebselement bevorzugt als rotierende Welle ausgebildet. Das Antriebselement kann je nach Art des Handstücks 8 weitere oder alternative Bauteile aufweisen, so zum Beispiel eine oder mehrere zusätzliche rotierende oder schwingende Wellen, ein oder mehrere Zahnräder oder Getriebe, einen Motor, ein pneumatisch betreibbares Laufrad, einen Schwingungserreger usw. Das dentale Handstück weist am Ende des Griffteils 9 einen Anschluß 15 auf, über den das Kopfteil 10 mit für den Antrieb notwendigen Fluiden oder Medien (z.B. Wasser, Druckluft, elektrischer Strom, etc.) versorgt werden kann.

Die erfindungsgemäße Beleuchtungsvorrichtung kann beispielsweise auf zweierlei Weise eingesetzt werden: singulär oder ringförmig.

Gemäß Figur 4 erfolgt die Beleuchtung 16 singulär, nämlich dadurch, dass in dem dem Kopfteil 10 zugewandten Ende des Griffteils 9 nur eine einzige erfindungsgemäße Beleuchtungsvorrichtung 1 integriert ist und somit die Abgabe eines gebündelten Lichtstrahles auf den Arbeitsbereich einer Behandlungsstelle bewirkt wird. Die Beleuchtungsvorrichtung 1 befindet sich dabei in einer Lichtabgabeöffnung der Außenhülse des Handstücks 8.

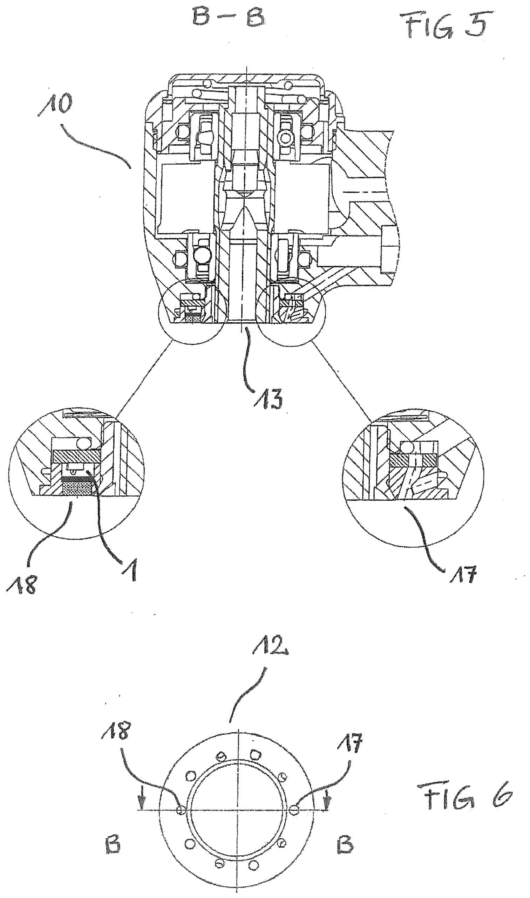

Gemäß den Figuren 5 und 6 erfolgt die Beleuchtung 18 ringförmig mittels eines ringförmigen Fluid- und Strahlungsabgabeendes 12, durch das sowohl Strahlung (z.B. Licht) als auch Fluid (z.B. Wasser, Druckluft, etc.) in den Arbeitsbereich einer Behandlungsstelle eingebracht werden können.

Figur 5 zeigt im Schnittbild das Kopfende 10 des dentalen Instrumentes 8, in dem mittig die Werkzeugöffnung 13 angeordnet ist. In diesem Schnittbild befindet sich links der Werkzeugöffnung 13 eine Strahlungsabgabeöffnung 18, in der die erfindungsgemäße Beleuchtungsvorrichtung 1 aufgenommen ist. Rechts der Werkzeugöffnung 13 befindet sich eine Fluidabgabeöffnung 17 in die mehrere Fluidkanäle einmünden, um unterschiedliche Fluide (z.B. Wasser und Druckluft) einzeln oder gemischt abgeben zu können. Aus Figur 6 ist ersichtlich, dass auf dem ringförmigen Fluid- und Strahlungsabgabeende 12 Strahlungsabgabeöffnungen 18 und Fluidabgabeöffhungen 17 alternierend angeordnet sind, um eine gewisse Gleichmäßigkeit der Strahlungs- bzw. Fluid-Abgabe zu erreichen.

Eine ausführliche Beschreibung des dentalen Instrumentes mit ringförmiger Beleuchtung ist in der WO 2013011075 offenbart.

### Bezugszeichenliste

- 1: Beleuchtungsvorrichtung (gemäß der Erfindung)
- 2: Substrat
- 3: Leuchtelement
- 4: Abstandhalter
- 5: Optisches Element (z.B. Linse)
- 6: Luftraum
- 7: Außenraum
- s: Außendurchmesser Abstandhalter
- t: Durchmesser Öffnung
- u: Höhe Abstandhalter
- v: Höhe Luftraum
- w: Linsenwölbung Innen
- x: Linsenwölbung Außen
- y: Linsenwölbung Innen bis Oberkante Abstandhalter
- z: Durchmesser obere Außenkante Schweißnut/Lötnut
- 8: dentales Instrument bzw. Handstück
- 9: Griffteil
- 10: Instrumentenkopf bzw. Kopfteil
- 11: Drucktaster
- 12: ringförmiges Fluid- und Strahlungsabgabeende
- 13: Werkzeugöffnung
- 14: Werkzeug
- 15: Anschluß
- 16: singuläre Beleuchtung
- 17: Fluidabgabeöffhung
- 18: Strahlungsabgabeöffnung

## Patentansprüche

1. Beleuchtungsvorrichtung (1) aufweisend
- ein Substrat (2),
- einen nicht transparenten Abstandhalter (4), welcher mit dem Substrat (2) hermetisch dicht verbunden ist,
- eine dem Substrat (2) gegenüberliegende Öffnung in dem Abstandhalter (4), und
- ein unter dem Abstandhalter (4) und unter der Öffnung befindliches Leuchtelement (3), wobei elektrische Anschlüsse des Leuchtelements (3) hermetisch dicht durch das Substrat (2) geführt sind,
wobei die Öffnung des Abstandhalters (4) mit einem optischen Element (5) aus einem Glasmaterial, dessen Volumen mindestens einen Leuchtstoff aufweist und als solches einen lumineszierenden Glasverbundwerkstoff darstellt, hermetisch dicht verschlossen ist,
**dadurch gekennzeichnet,**
**dass** der Glasverbundwerkstoff Füllmaterialien zur Einstellung einer Viskosität einer Glasmatrix bei einer Herstellung der Beleuchtungsvorrichtung (1) enthält.

2. Beleuchtungsvorrichtung (1) nach Anspruch 1,
**dadurch gekennzeichnet,**
**dass** der Glasverbundwerkstoff einen homogenen Leuchtstoff-Glas-Verbundwerkstoff darstellt,
**dass** eine homogen verteilte Einzel- oder Gesamtleuchtstoffkonzentration 5 bis 40 Vol.%, vorzugsweise zwischen 10 und 25 Vol.% aufweist, und
**dass** der Leuchtstoff einen Granat und/oder ein Nitrid/Oxynitrid und/oder ein Oxid, vorzugsweise YAG: Ce³⁺, LuAG: Ce³⁺, und/oder Y₂O₃:Eu³⁺ aufweist.

3. Beleuchtungsvorrichtung (1) nach einem der vorhergehenden Ansprüche,
**dadurch gekennzeichnet,**
**dass** der Abstandhalter (4) in Form einer Metallkappe gebildet ist, und
**dass** das Substrat (2) und der Abstandhalter (4) hermetisch verlötet, verschweißt, vorzugsweise laserverschweißt sind.

4. Beleuchtungsvorrichtung (1) nach einem der vorhergehenden Ansprüche,
**dadurch gekennzeichnet,**
**dass** das Substrat (2) aus Keramik oder Keramik-PCB gebildet ist.

5. Beleuchtungsvorrichtung (1) nach einem der vorhergehenden Ansprüche,
**dadurch gekennzeichnet,**
**dass** zwischen dem Glasverbundwerkstoff und dem Substrat (2) ein Luftraum (6) gebildet ist, der das Leuchtelement (3) enthält.

6. Beleuchtungsvorrichtung (1) nach einem der vorhergehenden Ansprüche,
**dadurch gekennzeichnet,**
**dass** das Leuchtelement (3) auf einem blau emittierenden LED Chip basiert.

7. Beleuchtungsvorrichtung (1) nach einem der vorherigen Ansprüche,
**dadurch gekennzeichnet,**
**dass** der Glasverbundwerkstoff lichtstreuende Zusatzstoffe enthält, und dass die lichtstreuenden Zusatzstoffe durch Al₂O₃ und/oder TiO₂ gegeben sind.

8. Beleuchtungsvorrichtung (1) nach einem der vorherigen Ansprüche,
**dadurch gekennzeichnet,**
**dass** der Abstandhalter (4) und der Glasverbundwerkstoff hermetisch durch Verschmelzung unter Schutzgas bei ca. 1000°C verbunden sind.

9. Beleuchtungsvorrichtung (1) nach einem der vorherigen Ansprüche,
**dadurch gekennzeichnet,**
**dass** der Glasverbundwerkstoff weitere Zusatzstoffe enthält, die bei einer Herstellung der Beleuchtungsvorrichtung (1) eine Trennung von Leuchtstoffteilchen von der Glasmatrix verhindern.

10. Beleuchtungsvorrichtung (1) nach einem der vorherigen Ansprüche,
**dadurch gekennzeichnet,**
**dass** die elektrischen Anschlüsse des Leuchtelements (3) hermetisch dicht auf dessen Unterseite liegen und durch das Substrat (2) mittels einer Glasdurchführung oder einer metallischen Durchkontaktierung ebenso hermetisch nach Außen geführt sind.

11. Beleuchtungsvorrichtung (1) nach einem der vorherigen Ansprüche,
**dadurch gekennzeichnet,**
**dass** eine Dicke des Glasverbundwerkstoffes zwischen 0,2 und 1,0 mm, vorzugsweise zwischen 0,4 und 0,8 mm, beträgt.

12. Beleuchtungsvorrichtung (1) nach einem der vorherigen Ansprüche,
**dadurch gekennzeichnet,**
**dass** das optische Element (5) aus lumineszierendem Glasverbundwerkstoff eine einseitige oder eine beidseitige Krümmung aufweist.

13. Verfahren zur Herstellung einer Beleuchtungsvorrichtung (1) gemäß den vorhergehenden Ansprüchen 1 bis 12,
umfassend folgende Schritte:
- Herstellung einer hermetischen Verbindung zwischen einem Abstandhalter (4) und einem optischen Element (5):
Variante 1:
A) Erzeugen eines stabförmigen Glaskörpers durch Schmelzen eines Glaspulvers mit darin homogen eingemischten Leuchtstoffteilchen und Füllmaterialien zur Einstellung einer Viskosität einer Glasmatrix bei der Herstellung der Beleuchtungsvorrichtung (1);
B) Abtrennen einer Glasscheibe (5) in gewünschter Dicke von dem Glaskörper;
C) Einlegen der Glasscheibe (5) in eine Öffnung des Abstandhalters (4); und
D) hermetisches Verschmelzen des Abstandhalters (4) und der Glasscheibe (5) im Bereich der Öffnung des Abstandhalters (4) bei ca. 1000°C unter Schutzgas;
Variante 2:
a) Pressen des Glaspulvers mit den darin homogen eingemischten Leuchtstoffteilchen und den Füllmaterialien zu einer Tablette (5);
b) Einlegen der Tablette (5) in die Öffnung des Abstandhalters (4); und
c) hermetisches Verschmelzen des Abstandhalters (4) und der Tablette (5) im Bereich der Öffnung des Abstandhalters (4) bei ca. 1000°C unter Schutzgas;
- Anbringen eines Leuchtelements (3) auf dem Substrat (2) und hermetisch dichtes Führen von elektrischen Anschlüssen des Leuchtelements (3) durch das Substrat (2) mittels einer Glasdurchführung oder einer metallischen Durchkontaktierung; und
- hermetisch dichtes Verbinden des Substrats (2) mit dem Abstandhalter (4) durch ein Verlöten oder Verschweißen, vorzugsweise Laserschweißen.

14. Verfahren nach Anspruch 13,
**dadurch gekennzeichnet,**
**dass** das optische Element (5) während des Schrittes des Verschmelzens durch geeignete Formelemente in eine bestimmte Form gebracht wird.

15. Medizinisches, insbesondere dentales Hand- oder Winkelstück,
**dadurch gekennzeichnet,**
**dass** dieses eine Beleuchtungsvorrichtung nach einem der Ansprüche 1 bis 12 aufweist.

## Claims

1. Illuminating device (1) comprising
- a substrate (2),
- a non-transparent spacer (4) connected to the substrate (2) in a hermetically sealed manner,
- an opening in the spacer (4) opposite to the substrate (2), and
- a lighting element (3) located below the spacer (4) and below the opening, wherein electrical connections of the lighting element (3) are passed through the substrate (2) in a hermetically sealed manner,
wherein the opening of the spacer (4) is closed in a hermetically sealed manner with an optical element (5) made of a glass material, the volume of which comprises at least one luminescent substance and as such is a luminescent glass composite material,
**characterized in that**
the glass composite material contains filling materials for adjusting a viscosity of a glass matrix during production of the illuminating device (1).

2. Illuminating device (1) according to Claim 1,
**characterized in that**
the glass composite material is a homogeneous luminescent substance-glass composite material,
the homogeneously distributed individual or total luminescent concentration is 5 to 40 vol.%, preferably between 10 and 25 vol.%, and
the luminescent substance comprises a garnet and/or a nitride/oxynitride and/or an oxide, preferably YAG: Ce³⁺, LuAG: Ce³⁺, and/or Y₂O₃:EU³⁺.

3. Illuminating device (1) according to any one of the preceding claims,
**characterized in that**
the spacer (4) is formed in the shape of a metal cap, and the substrate (2) and spacer (4) are hermetically soldered, welded, preferably laser welded.

4. Illuminating device (1) according to any one of the preceding claims,
**characterized in that**
the substrate (2) is made of ceramic or ceramic PCB.

5. Illuminating device (1) according to any one of the preceding claims,
**characterized in that**
an air space (6) containing the lighting element (3) is formed between the glass composite material and the substrate (2).

6. Illuminating device (1) according to any one of the preceding claims, **characterized in that**
the lighting element (3) is based on a blue-emitting LED chip.

7. Illuminating device (1) according to any one of the preceding claims,
**characterized in that**
the glass composite material contains light-scattering additives and that the light-scattering additives are provided by Al₂O₃ and/or TiO₂.

8. Illuminating device (1) according to any one of the preceding claims,
**characterized in that**
the spacer (4) and the glass composite material are hermetically connected by fusing under protective gas at approximately 1000°C.

9. Illuminating device (1) according to any one of the preceding claims,
**characterized in that**
the glass composite material contains additional additives which prevent separation of the luminescent material particles from the glass matrix during production of the illuminating device (1).

10. Illuminating device (1) according to any one of the preceding claims,
**characterized in that**
the electrical connections of the lighting element (3) lie hermetically sealed on its underside and are also hermetically guided through the substrate (2) to the outside through a glass feed-through or through a metallic through-connection.

11. Illuminating device (1) according to any one of the preceding claims,
**characterized in that**
the thickness of the glass composite material is between 0.2 and 1.0 mm, preferably between 0.4 and 0.8 mm.

12. Illuminating device (1) according to any one of the preceding claims,
**characterized in that**
the optical element (5) made of the luminescent glass composite material comprises a curvature on one or both sides.

13. Method for producing an illuminating device (1) according to the preceding Claims 1 to 12,
comprising the following steps:
- producing a hermetic connection between a spacer (4) and an optical element (5):
Variant 1:
A) producing a rod-shaped glass body by melting a glass powder comprising homogeneously incorporated luminescent material particles and filling materials for adjusting a viscosity of a glass matrix during production of the illuminating device (1);
B) separating a glass pane (5) having the desired thickness from the glass body;
C) inserting the glass pane (5) into an opening of the spacer (4); and
D) hermetically fusing the spacer (4) and the glass pane (5) in the region of the opening of the spacer (4) at approximately 1000°C under protective gas;
Variant 2:
a) pressing the glass powder with the homogeneously incorporated luminescent material particles and the filling materials into a tablet (5);
b) inserting the tablet (5) into the opening of the spacer (4); and
c) hermetically fusing the spacer (4) and the tablet (5) in the region of the opening of the spacer (4) at approximately 1000°C under protective gas;
- attaching a lighting element (3) to the substrate (2) and guiding electrical connections of the lighting element (3) through the substrate (2) in a hermetically sealed manner by means of a glass feed-through or a metallic through-connection; and
- connecting the substrate (2) to the spacer (4) in a hermetically sealed manner by soldering or welding, preferably laser welding.

14. Method according to Claim 13,
**characterized in that**
the optical element (5) is brought into a specific shape during the fusing step using suitable mold elements.

15. Medical, in particular dental, handpiece or contra-angle handpiece,
**characterized in that**
it comprises an illuminating device according to any one of Claims 1 to 12.

## Revendications

1. Dispositif d'éclairage (1) présentant :
- un substrat (2),
- un organe d'espacement (4) non transparent raccordé hermétiquement au substrat (2),
- une ouverture ménagée dans l'organe d'espacement (4) à l'opposé du substrat (2), et
- un élément luminescent (3) situé sous l'organe d'espacement (4) et sous l'ouverture, les bornes électriques de l'élément luminescent (3) passant hermétiquement à travers le substrat (2) ;
ladite ouverture de l'organe d'espacement (4) étant obturée hermétiquement par un élément optique (5) se composant d'un matériau à base de verre dont le volume présente au moins une substance luminophore et, en tant que tel, représente un matériau composite à base de verre luminescent,
**caractérisé en ce que**
le matériau composite à base de verre contient des matières de charge permettant d'adapter la viscosité d'une matrice vitreuse lors de la fabrication du dispositif d'éclairage (1).

2. Dispositif d'éclairage (1) selon la revendication 1, **caractérisé en ce que**
le matériau composite à base de verre représente un matériau composite homogène à base de substance luminophore et de verre, une concentration en substance luminophore individuelle ou totale distribuée de façon homogène va de 5 à 40 % en volume, de préférence de 10 à 25 % en volume, et
la substance luminophore présente un grenat et/ou un nitrure/oxynitrure et/ou un oxyde, de préférence un YAG: Ce³⁺, un LuAG : Ce³⁺, et/ou un Y₂O₃ : Eu³⁺.

3. Dispositif d'éclairage (1) selon l'une des revendications précédentes,
**caractérisé en ce que**
l'organe d'espacement (4) présente la forme d'un capuchon métallique, et
le substrat (2) et l'organe d'espacement (4) sont brasés ou soudés hermétiquement, de préférence soudés au laser.

4. Dispositif d'éclairage (1) selon l'une des revendications précédentes,
**caractérisé en ce que**
le substrat (2) est formé de céramique ou de carte de circuit imprimé à base de céramique.

5. Dispositif d'éclairage (1) selon l'une des revendications précédentes,
**caractérisé en ce que**,
il est formé un espace d'air (6) contenant l'élément luminescent (3) entre le matériau composite à base de verre et le substrat (2).

6. Dispositif d'éclairage (1) selon l'une des revendications précédentes, **caractérisé en ce que**
l'élément luminescent (3) repose sur une puce LED émettant une lumière bleue.

7. Dispositif d'éclairage (1) selon l'une des revendications précédentes,
**caractérisé en ce que** le matériau composite à base de verre contient des additifs diffusant la lumière et les additifs diffusant la lumière sont fournis par Al₂O₃ et/ou TiO₂.

8. Dispositif d'éclairage (1) selon l'une des revendications précédentes,
**caractérisé en ce que**
l'organe d'espacement (4) et le matériau composite à base de verre sont raccordés hermétiquement par fusion sous atmosphère protectrice à env. 1000 °C.

9. Dispositif d'éclairage (1) selon l'une des revendications précédentes,
**caractérisé en ce que**
le matériau composite à base de verre contient d'autres additifs qui empêchent une séparation entre les particules de substance luminophore et la matrice vitreuse lors de la fabrication du dispositif d'éclairage (1).

10. Dispositif d'éclairage (1) selon l'une des revendications précédentes,
**caractérisé en ce que**
les bornes électriques de l'élément luminescent (3) reposent hermétiquement sur sa face inférieure et sont guidées vers l'extérieur, de manière également hermétique, en traversant le substrat (2) au moyen d'une traversée de verre ou d'un trou d'interconnexion métallique.

11. Dispositif d'éclairage (1) selon l'une des revendications précédentes,
**caractérisé en ce que**
l'épaisseur du matériau composite à base de verre se situe entre 0,2 et 1,0 mm, de préférence entre 0,4 et 0,8 mm.

12. Dispositif d'éclairage (1) selon l'une des revendications précédentes,
**caractérisé en ce que**
l'élément optique (5) à base de matériau composite à base de verre luminescent présente une courbure sur une face ou sur les deux.

13. Procédé de fabrication d'un dispositif d'éclairage (1) selon les revendications précédentes 1 à 12,
comprenant les étapes suivantes :
- fabrication d'un raccord hermétique entre l'organe d'espacement (4) et un élément optique (5) :
variante 1 :
A) production d'un corps de verre en forme de tige par fusion d'une poudre de verre dans laquelle sont réparties de façon homogène des particules de substance luminophore et des matières de charge permettant d'adapter la viscosité d'une matrice vitreuse lors de la fabrication du dispositif d'éclairage (1) ;
B) sectionnement d'un disque de verre (5) d'une épaisseur voulue à partir du corps de verre ;
C) insertion du disque de verre (5) dans une ouverture de l'organe d'espacement (4) ; et
D) fusion hermétique de l'organe d'espacement (4) et du disque de verre (5) dans le secteur de l'ouverture de l'organe d'espacement (4) à env. 1000 °C sous atmosphère protectrice ;
variante 2 :
a) compression de la poudre de verre dans laquelle sont réparties de façon homogène les particules de substance luminophore et les matières de charge pour former un comprimé (5) ;
b) insertion du comprimé (5) dans l'ouverture de l'organe d'espacement (4) ; et
c) fusion hermétique de l'organe d'espacement (4) et du comprimé (5) dans le secteur de l'ouverture de l'organe d'espacement (4) à env. 1000 °C sous atmosphère protectrice ;
- pose d'un élément luminescent (3) sur le substrat (2) et passage hermétique des bornes électriques de l'élément luminescent (3) à travers le substrat (2) au moyen d'une traversée de verre ou d'un trou d'interconnexion métallique ; et
- raccordement hermétique du substrat (2) à l'organe d'espacement (4) par brasage ou soudage, de préférence par soudage laser.

14. Procédé selon la revendication 13,
**caractérisé en ce que**,
pendant l'étape de fusion hermétique, l'élément optique (5) est façonné selon une forme déterminée à l'aide d'éléments de moulage appropriés.

15. Instrument médical, notamment pièce à main ou contre-angle dentaire,
**caractérisé en ce que**
ledit instrument présente un dispositif d'éclairage selon l'une des revendications 1 à 12.
